# EUROPEAN PATENT SPECIFICATION

(11) **EP 4 363 854 B1**
(45) Date of publication and mention of the grant of the patent: **24.06.2026**
(21) Application number: 22744516.0
(22) Date of filing: 30.06.2022
(51) Int. Cl.: G01N 33/575, C12Q 1/6804

(54) **METHOD FOR IDENTIFYING AND/OR QUANTIFYING DNA DAMAGES IN PROXIMITY OF TRANSCRIBED GENOMIC LOCI**
VERFAHREN ZUR IDENTIFIZIERUNG UND/ODER QUANTIFIZIERUNG DNA-SCHÄDEN IN DER NÄHE TRANSKRIBIERTER GENOMISCHEN LOCI
PROCÉDÉ D'IDENTIFICATION ET/OU DE QUANTIFICATION DE DOMMAGES À L'ADN À LA PROXIMITÉ DE LOCUS GÉNOMIQUES TRANSCRITS

(30) Priority: 01.07.2021 IT 202100017414
(43) Date of publication of application: 08.05.2024
(73) Proprietor: Fondazione Istituto Italiano di Tecnologia, 16163 Genova (IT)
(72) Inventor: CAMPANER, Stefano, 16163 Genova (IT); CURTI, Laura, 16163 Genova (IT)
(74) Representative: Studio Torta S.p.A.
(86) International application number: PCT/IB2022/056112
(87) International publication number: WO 2023/275818

(56) References cited:
- US-A1- 2020 248 271
- SAMAH W AWWAD ET AL: "NELF-E is recruited to DNA double-strand break sites to promote transcriptional repression and repair", EMBO REPORTS, NATURE PUBLISHING GROUP, LONDON, GB, vol. 18, no. 5, 23 March 2017 (2017-03-23), pages 745 - 764, XP072236629, ISSN: 1469-221X, DOI: 10.15252/EMBR.201643191
- JANA SUCHÁNKOVÁ ET AL: "Distinct kinetics of DNA repair protein accumulation at DNA lesions and cell cycle-dependent formation of [gamma]H2AX- and NBS1-positive repair foci", BIOLOGY OF THE CELL, ELSEVIER, PARIS, FR, vol. 107, no. 12, 17 November 2015 (2015-11-17), pages 440 - 454, XP071518930, ISSN: 0248-4900, DOI: 10.1111/BOC.201500050
- MARTIN OLGA A. ET AL: "[gamma]-H2AX in Cancer Cells: A Potential Biomarker for Cancer Diagnostics, Prediction and Recurrence", CELL CYCLE, vol. 5, no. 24, 28 November 2006 (2006-11-28), US, pages 2909 - 2913, XP055892666, ISSN: 1538-4101, DOI: 10.4161/cc.5.24.3569
- SIDDIQUI MOHAMMAD SABBIR ET AL: "Persistent [gamma]H2AX: A promising molecular marker of DNA damage and aging", MUTATION RESEARCH. REVIEWS IN MUTATION RESEARCH, vol. 766, 1 October 2015 (2015-10-01), NL, pages 1 - 19, XP055892623, ISSN: 1383-5742, DOI: 10.1016/j.mrrev.2015.07.001
- NAIDOO KALNISHA ET AL: "Evaluation of CDK12 Protein Expression as a Potential Novel Biomarker for DNA Damage Response-Targeted Therapies in Breast Cancer", vol. 17, no. 1, 1 January 2018 (2018-01-01), US, pages 306 - 315, XP055892570, ISSN: 1535-7163, Retrieved from the Internet <URL:https://watermark.silverchair.com/306.pdf?token=AQECAHi208BE49Ooan9kkhW_Ercy7Dm3ZL_9Cf3qfKAc485ysgAAA9AwggPMBgkqhkiG9w0BBwagggO9MIIDuQIBADCCA7IGCSqGSIb3DQEHATAeBglghkgBZQMEAS4wEQQM1HUKN1K3mn0desLyAgEQgIIDgwIgPSiJe2fg9Y2lWknE9A_yMBCvyTQ5teY8g9erkSBY7uqBVh2gyPvjPorA88SQnEwbDgKNHzmNKKJ633T9Dpc2ZzC7nyV0> DOI: 10.1158/1535-7163.MCT-17-0760
- PILAROVA KVETA ET AL: "CDK12: cellular functions and therapeutic potential of versatile player in cancer", vol. 2, no. 1, 1 March 2020 (2020-03-01), pages 1, XP055892584, Retrieved from the Internet <URL:https://watermark.silverchair.com/zcaa003.pdf?token=AQECAHi208BE49Ooan9kkhW_Ercy7Dm3ZL_9Cf3qfKAc485ysgAAAugwggLkBgkqhkiG9w0BBwagggLVMIIC0QIBADCCAsoGCSqGSIb3DQEHATAeBglghkgBZQMEAS4wEQQM-AxsvMYmS9buqJBOAgEQgIICm6yZNsyA8JWYiZZJzIcf9PuJhDSeGU3kVpgNJSBx2edtc5jbbUzqGWeOFSNEiydUKEaC8Gj-VYKo4NW762fbjv-I15yG> DOI: 10.1093/narcan/zcaa003
- JALILI ROXANA ET AL: "Streamlined circular proximity ligation assay provides high stringency and compatibility with low-affinity antibodies", vol. 115, no. 5, 30 January 2018 (2018-01-30), pages E925 - E933, XP055800525, ISSN: 0027-8424, Retrieved from the Internet <URL:https://www.pnas.org/content/pnas/115/5/E925.full.pdf> DOI: 10.1073/pnas.1718283115
- LIANG S. ET AL: "cdk12...", 18 June 2020 (2020-06-18), XP055892617, Retrieved from the Internet <URL:https://www.ncbi.nlm.nih.gov/pmc/articles/PMC7349380/pdf/cells-09-01483.pdf> [retrieved on 20220216]
- CHIRACKAL MANAVALAN ANIL PAUL ET AL: "CDK12 controls G1/S progression by regulating RNAPII processivity at core DNA replication genes", vol. 20, no. 9, 25 July 2019 (2019-07-25), GB, XP055892610, ISSN: 1469-221X, Retrieved from the Internet <URL:https://onlinelibrary.wiley.com/doi/full-xml/10.15252/embr.201847592> DOI: 10.15252/embr.201847592

## Description

### Technical Field of the Invention

The present invention relates to a method for identifying and/or quantifying in a sample DNA damage events associated to actively transcribed genomic loci, comprising detecting the CDK12 protein in proximity of the aforesaid genomic loci.

### Prior Art

Genomic instability is a hallmark of tumour cells (Hanahan and Weinberg, 2011). This characteristic is used for clinical classification, but is also considered a key condition that can be exploited for targeted therapies. Therefore, molecular assays are needed that allow the identification and classification of processes that control the stability of the genome both for diagnostics and prognostics in the clinic and for the development of targeted therapies.

Several processes have been causally linked to genomic instability in tumour cells, such as mutations in oncosuppressor care taker genes, defects in telomere maintenance and protection, altered oxidative metabolism and oncogene-induced replicative stress.

Each of these processes affects the stability of the regions of the genome that have distinctive structural, functional and operational properties. Moreover, each of these processes has specific consequences in shaping the genome of tumour cells during cancer progression and in sensitising tumour cells to targeted therapies that exploit selective deficiencies in genome maintenance processes (Zhu et al. , 2020).

The knowledge of which genome stability process is active or altered in tumour cells during tumour progression or in association with different therapeutic regimes is therefore essential for the clinical classification of tumours and the evaluation of therapeutic regimes, to monitor ongoing therapeutic treatment and in clinical and preclinical studies (Cleary et al., 2020).

It is therefore essential to identify methods that allow a scalable and quantitative assessment of transductional pathways linked to genome integrity.

Over the years, several assays have been developed to assess the general activation of DNA damage response pathways or the detection of DNA breaks, based on antibody detection of the phosphorylated form of the histone protein H2Ax or on the nuclear staining pattern of proteins that are effectors of DNA damage response pathways (Cleary et al., 2020). In addition, methods are available for assessing the ability of the tumour cells to repair certain types of DNA damage, such as methods for assessing microsatellite instability (Hissong et al., 2018).

However, there is still a lack of methods that allow molecular evaluation of the transductional pathways linked to oncogene-induced replicative stress (hereinafter: OI-RS): a process driven by the alteration of DNA replication due to the aberrant ignition and activity of the replication machinery, which in turn promotes collisions of replicating DNA with transcriptional complexes. This leads to the accumulation of DNA breaks that (1) lead to genomic alterations (translocation, insertions and small amplifications (2) expose/sensitise cells to the action of specific genotoxic drugs.

Experimentally, the collision of DNA replication and transcription and the generation of DNA breaks can be inferred from the independent evaluation of (i) DNA replication dynamics (either by DNA combing techniques, multilabel immunofluorescence, or NGS-based analysis of replication dynamics, (ii) transcription evaluation (by NGS-based nascent RNA analysis and RNA-pol2 dynamics), and (iii) DNA damage evaluation (either by direct evaluation of DNA breaks by comet assay, NGS-based methods, and other methods, or by evaluation of DNA damage foci).

This process is cumbersome, and even though it is informative for investigational studies, it cannot be used for clinical evaluation of OI-RS, nor can it be scaled up for high-productivity, information-intensive analysis required for both screening campaigns and drug discovery projects. Similarly, the use of such assays in clinical application as diagnostics or evaluation of therapeutic regimes is inherently impractical.

Therefore, the lack of a single marker-based assay/method allowing the quantitative assessment of replicative stress characterised by damaged DNA proximal to transcribed genomic loci hinders and delays the clinical classification of tumour samples and the development of diagnostics and therapeutics.

O.A. Martin & W.M. Bonner (2006) discloses the formation of γH2AX (wherein its accumulation in cells appears as discrete nuclear foci when imaged by a laser scanning confocal microscopy) as a sensitive marker for DNA damage.

M.S. Siddiqui et al (2015) teaches the role of γH2AX as recruiter for proteins that are involved in the process of DNA damage repair.

K. Naidoo et al (2018) discloses the potential use of CDK12 as a marker for stratification of breast cancer and for identifying patients who may benefit from a DDR (DNA damage repair)-targeted therapy.

K. Pilarova et al (2020) is a review paper disclosing the function of CDK12 i.e. in transcription of genes expressing proteins involved in DNA repair and in the regulation of genome stability.

R. Jalili et al (2018) discloses the circular proximity ligation assay as a highly sensitive method for detecting target analytes at the femtomolar level.

S. Liang et al (2020) is a review paper discussing the biological role of CDK12; i.e. in the regulation of the process of DNA damage response and maintenance of genomic stability.

S.W. Awwad et al (2017) teaches that NELF-E is a molecule that has a role in DNA damage response by promoting transcriptional silencing after double strand break induction.

J. Suchánková et al (2015) relates to the kinetics and spatiotemporal distribution of the accumulation of proteins (i.e. yH2AX and BRCA1) involved in the process of DNA damage repair at DNA lesions.

### Summary of the Invention

It is therefore an aim of the present invention to provide a method for identifying and/or quantifying in a sample the DNA damage events associated to actively transcribed genomic loci as defined in claim 1.

### Brief Description of the Figures

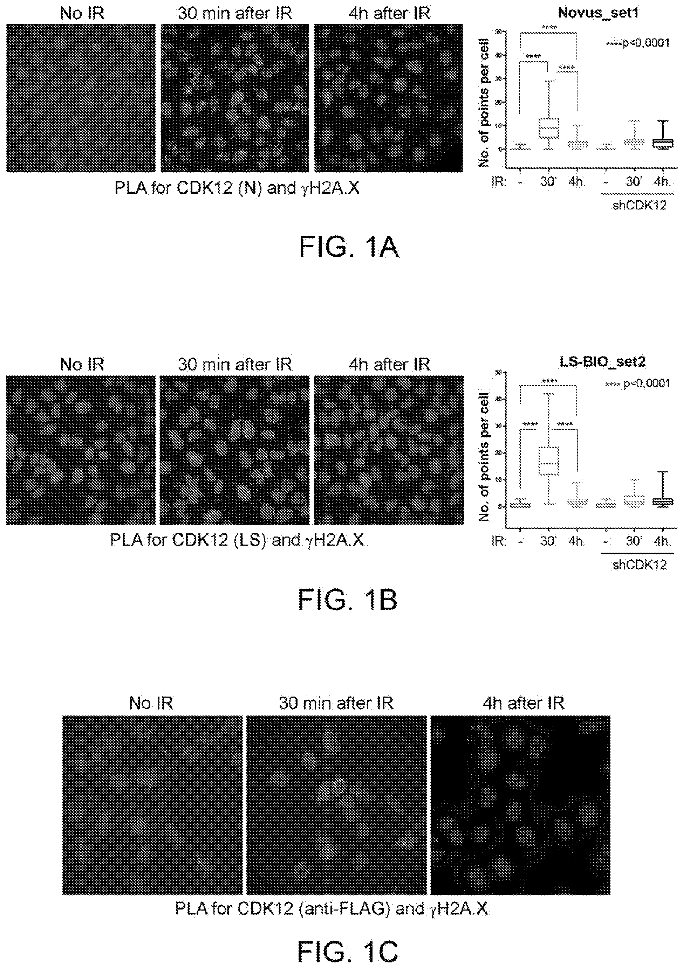

Figures 1A to 1C show the results relating to the proximity between CDK12 and DDR foci in X-ray irradiated cells. In particular, Figures 1A and 1B show the PLA test performed in U2OS cells, in the presence or absence of CDK12 silencing by shRNA. The cells were irradiated (IR: 10 Gy) and fixed at different times after irradiation (30 minutes or 4 hours). The PLA test was performed using two different antibodies against CDK12: anti-CDK12 # (Novus) (Fig. 1A) and anti-CDK12 # (LS-BIO) (Fig. 1B). To assess the presence of DDR foci, an antibody specific to the phosphorylated form of histone H2Ax (γH2Ax) was used. The graphs on the right represent the distribution of cells considering the number of nuclear PLA-foci counted in 300 cells for each condition. Figure 1C: Proximity Ligation Assay (PLA) performed on U2OS cells subjected to transient transfection in order to over-express FLAG-CDK12 protein.

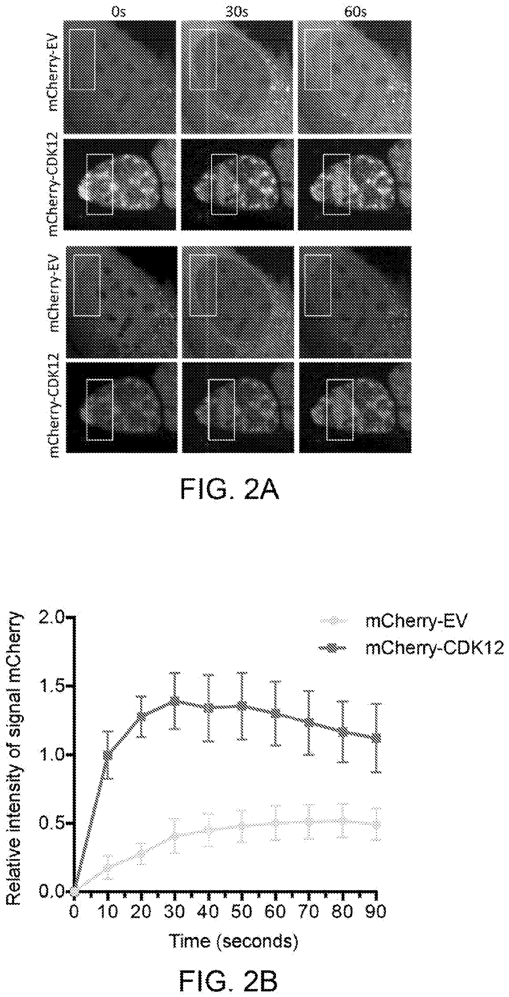

Figures 2A and 2B show how CDK12 is recruited to regions by DNA that are damaged by micro-irradiation by means of a laser beam. U2OS cells were transfected with the mCherry-CDK12 plasmid and then micro-irradiated with 10 iterations of a laser at 405 nm with 100% power, in order to generate DNA damage in localised nuclear regions. The cells were then subjected to microscopic analysis using time-lapse microscopy. The mCherry signal in the damaged regions was subjected to quantitative analysis. For each condition, at least 30 cells were analysed. Figure 2A: representative images. Figure 2B: kinetic analysis of fluorescence recovery at damaged sites.

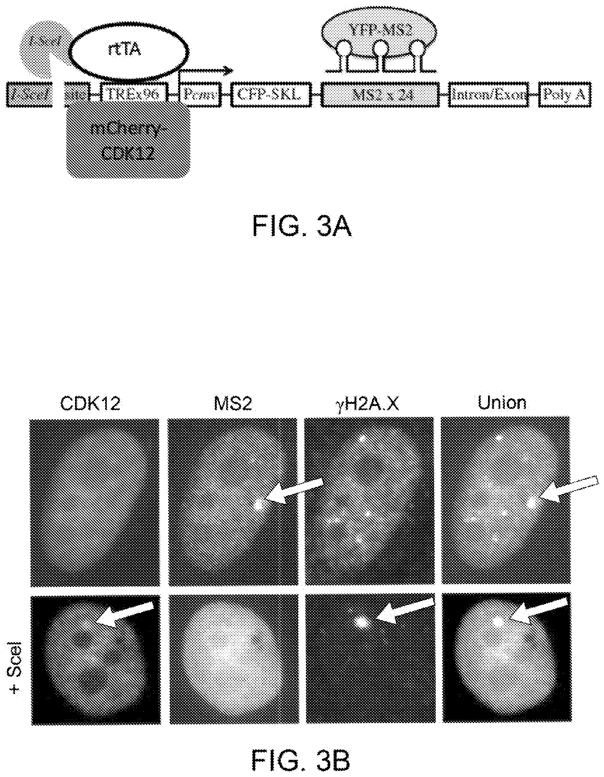

Figures 3A and 3B show how CDK12 is recruited to damaged DNA sites. U2OS-TRE-I-Sce1 cells were transfected with mCherry-CDK12, rtTA, YFP-MS2 and I-Sce1 plasmids, as indicated. The cells were treated with doxycycline to activate MS2 gene transcription, fixed with 4% PFA for 15 minutes and stained with antibodies specific to γH2A. X. Figure 3A: diagram of the reporter system and its fluorescent signals. Figure 3B: representative images of the nuclei, the arrows indicate the focal signals of DDR (γH2A. X), transcription (MS2) and CDK12 recruitment.

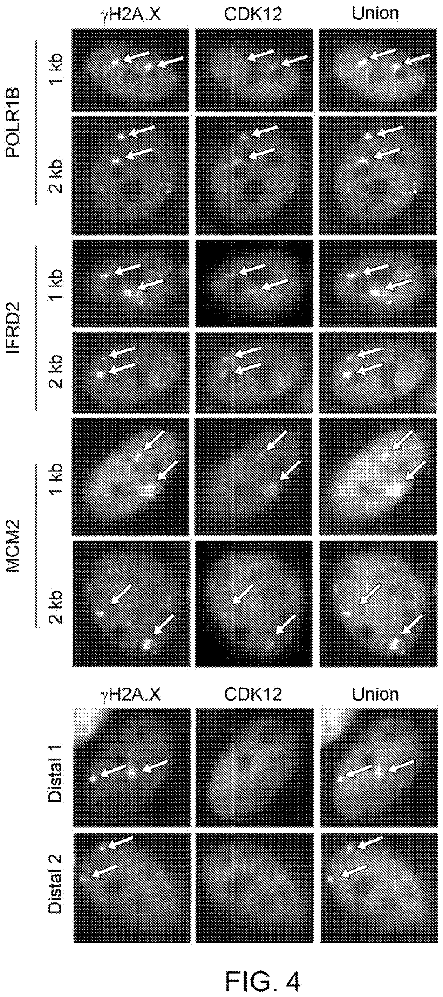

Figure 4 shows how CDK12 specifically localises in damaged regions of DNA that are proximal to transcribed gene promoters. RPE iCUT cells were transfected with mCherry-CDK12. CRISPR-Cas9 was activated for 16 hours by administration of doxycycline and SHIELD-1. Subsequently, the cells were transfected with Alt-R gRNA against regions positioned at 1 kb and 2 kb upstream of the TSS of the POLR1B, IFRD2, and MCM2 genes. 8 hours after transfection, the cells were fixed with 4% PFA and stained with antibodies specific to γH2A. X. The same experiment was performed using Alt-R gRNAs specific to two transcriptionally inactive genomic regions (distal 1 and distal 2). The arrows indicate the focal signals for DDR (YH2A. X) and CDK12.

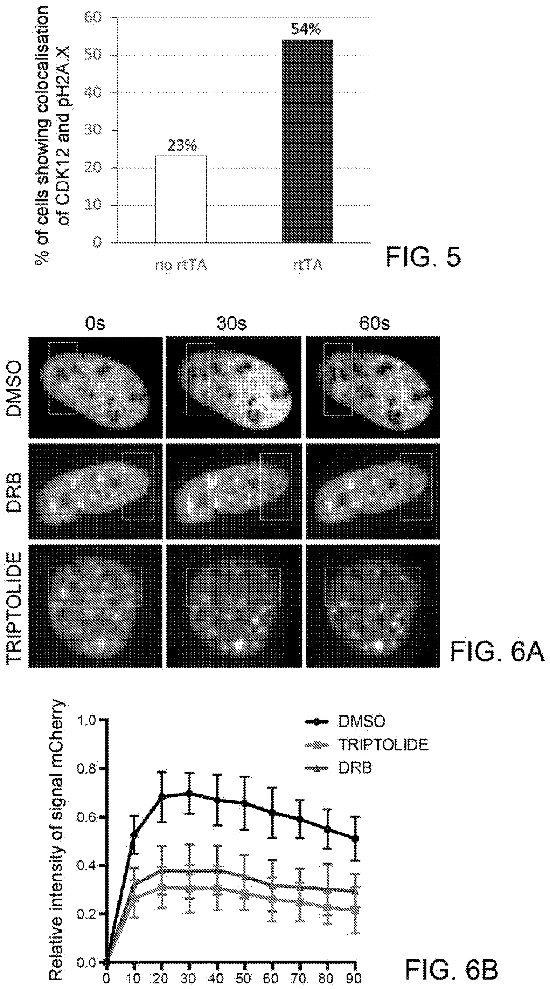

Figure 5 shows how the recruitment of CDK12 to DSBs proximal to the TRE-I reporter promoter is transcription-dependent. U2OS-TRE-I-SceI cells were transfected with plasmid vectors encoding mCherry-CDK12, MS2, SceI and with or without rtTA. rtTA was activated by administering doxycycline for 5 hours. Recruitment of CDK12 was assessed by immunofluorescence to determine colocalisation with foci of DDR (visualised with anti-γH2A. X antibody). The bar graph reports the number of cells showing co-localisation between CDK12 and γH2A. X (mean of two samples).
Figures 6A and 6B show how the recruitment of CDK12 to DDR sites is transcription-dependent. U2OS cells, transfected with mCherry-CDK12 plasmid, were first treated with DRB (100 uM), Triptolide (1 uM) or DMSO (mock) for 3 hours and then micro-irradiated by laser. Selected regions within the nucleus were micro-irradiated with 10 iterations of a laser at 405 nm with 100% power. The cells were subjected to quantitative analysis using time-lapse microscopy. Figure 6A: representative images used for the analysis shown in the graph reported in Figure 6B.

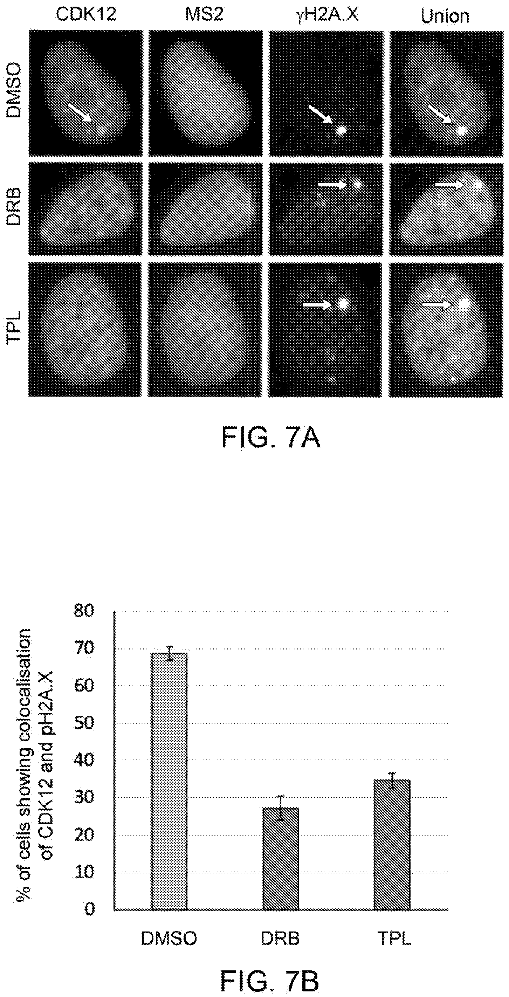

Figures 7A and 7B show how CDK12 recruitment at a DNA-damaged locus is transcriptionally dependent. U2OS-TRE-I-SceI cells were transfected with plasmid vectors encoding mCherry-CDK12, p-rtTA, YFP-MS2 or I-Sce1. The cells were treated with doxycycline, which is required for the induction of the rtTA transcription factor that activates the transcription of the MS2 reporter gene. RNA-pol2 activity was inhibited by DRB (100uM) or TRIPTOLIDE (TPL, 1uM) for 3 hours. The cells were fixed with 4% PFA for 15 minutes and immunostained for γH2A. X. The table in Figure 7B reports the percentage of cells showing co-localisation between CDK12 and γH2A. X (mean of two experiments).

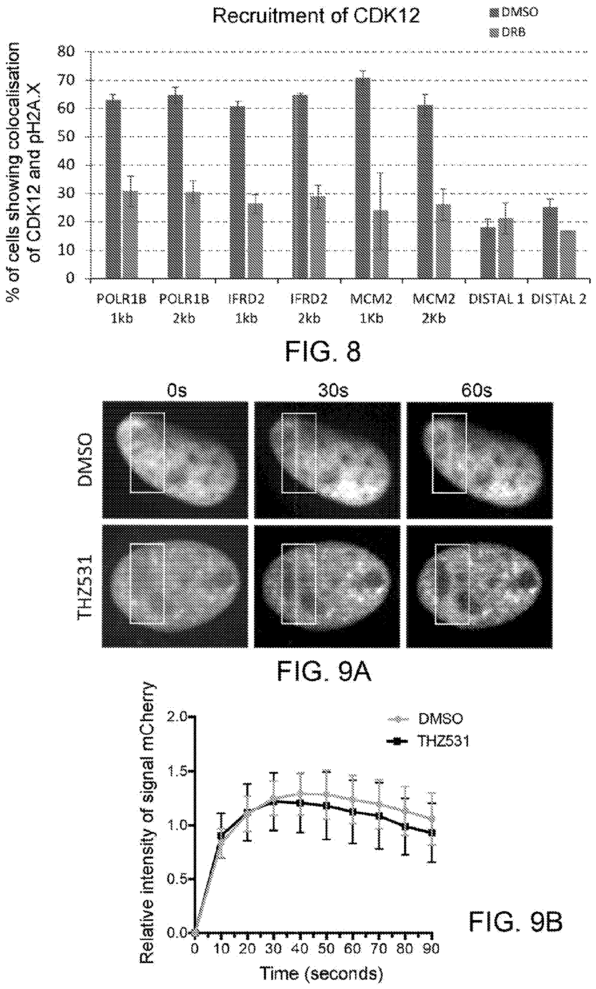

Figure 8 shows the selective recruitment of CDK12 to DSBs proximal to transcribed genomic loci. RPE iCUT cells, stably expressing inducible CRISPER-Cas9, were transfected with mCherry-CDK12. Cas9 was activated by doxycycline and SHIELD-1 for 16 hours. Cells were transfected with Alt-R gRNA against specific regions at 1 kb and 2 kb upstream of the TSS of three transcribed genes (POLR1B, IFRD2 and MCM2) and two transcriptionally inactive genomic regions (distal 1 and distal 2). 3 hours before harvesting, the cells were treated with DRB (100 uM). The bar graph reports the percentage of cells showing co-localisation of CDK12 and γH2A. X in the indicated genomic regions (mean of two independent experiments, 40 cells were considered for each region) .
Figures 9A and 9B show that the recruitment of CDK12 is independent of its catalytic activity. U2OS cells were transfected with the mCherry-CDK12 encoding plasmid and treated with THZ531 (100 nM) or DMSO for 24 hours. The cells were previously treated with BrdU for 24 hours to sensitise the DNA to breaks by laser. To generate localised DNA damage, selected regions within the nucleus were micro-irradiated with 10 iterations of a laser at 405 nm with 100% power. The cells were then subjected to time-lapse microscopic analysis. For each condition, at least 30 cells were considered. Figure 9A reports representative images used for the analysis shown in the graph of Figure 9B.

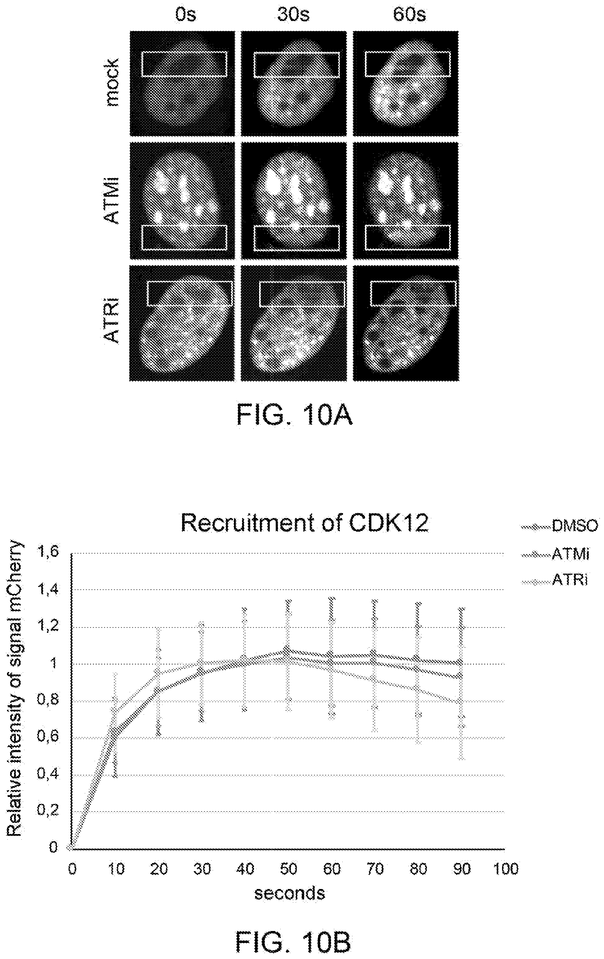

Figures 10A and 10B show that CDK12 recruitment at DDR sites is independent of ATM and ATR. U2OS cells were transfected with mCherry-CDK12 plasmid and pre-treated with KU-55933 (ATMi; 10 µM), VE-821 (ATRi; 10 µM) or DMSO (mock) for 2 hours prior to laser microirradiation. To generate localised DNA damage, selected regions within the nucleus were micro-irradiated with 10 iterations of a laser at 405 nm with 100% power. The cells were subjected to quantitative analysis by time-lapse microscopy. Figure 10A: representative images used for the analysis shown in the graph in Figure 10B.

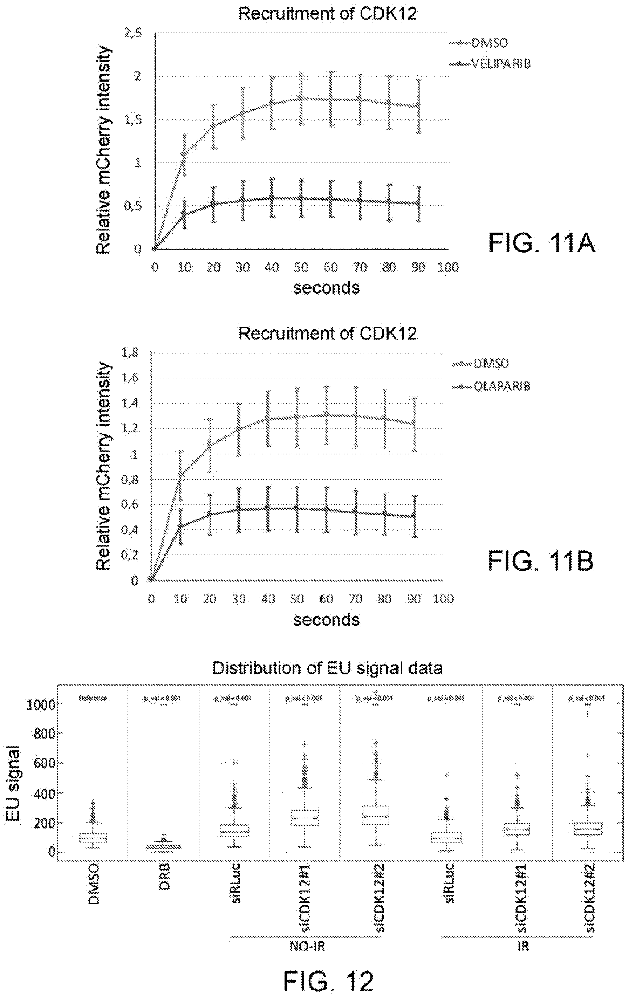

Figures 11A and 11B show how CDK12 recruitment to DDR sites depends on PARP1 activity. U2OS cells were transfected with mCherry-CDK12 plasmid and pre-treated with Olaparib (1 µM), Veliparib (10 µM) or DMSO (mock) for 1 hour before laser microirradiation. To generate localised DNA damage, selected regions within the nucleus were micro-irradiated with 10 iterations of a laser at 405 nm with 100% power. The cells were then subjected to quantitative analysis by time-lapse microscopy.
Figure 12 shows how CDK12 acts as a transcriptional repressor. Analysis of nascent RNA in U2OS cells transfected with one control siRNA and two different CDK12-targeting siRNAs. The cells were irradiated (10 Gy) and labelled with 5-EU for 20 minutes before harvesting. 5-EU incorporation was detected using Click-IT chemistry (Alexa Fluor 488). The box plot shows the EU signal intensity normalised for the control cells. The cells treated with DMSO and DRB were used as internal controls for the synthesis of nascent RNA.

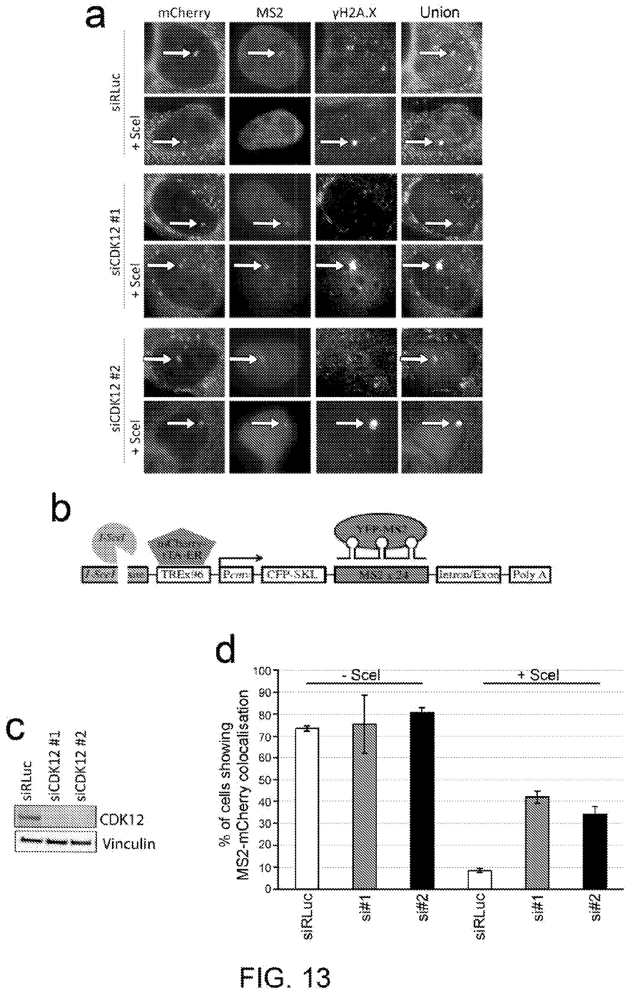

Figures 13A to 13D show how CDK12 is required to repress transcription at damaged DNA sites. U2OS-TRE-I-Sce1 cells were treated with control siRNA (siRLuc) or two different siRNAs against CDK12 (siCDK12 #1 and siCDK12 #2), and subsequently with pCherry-tTA-ER, YFP-MS2 and I-Sce1 plasmids. The fixed cells were immunostained for γH2A. X. (a) representative images (b) reporter system diagram (c) Western blot analysis showing the efficiency of CDK12 silencing. Vinculin (vin) was used as a control for normalisation. (d) Bar graph of the percentage of cells showing an active transcription (co-localisation of YFP-MS2 and pCherry-tTA-ER). The data shown are the mean of two independent experiments.

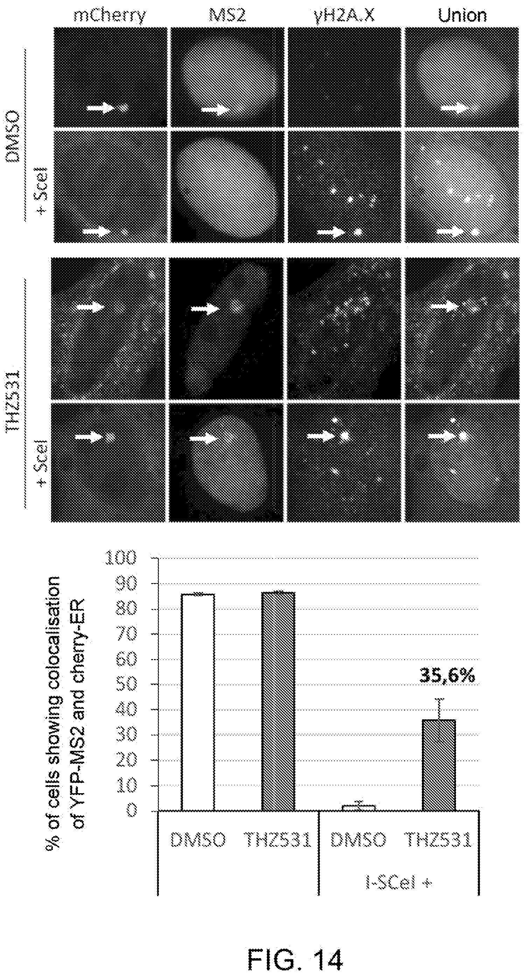

Figure 14 shows how CDK12 activity promotes transcriptional silencing in damaged genes. U2OS-TRE-I-Sce1 cells were transfected with pCherry-tTA-ER, YFP-MS2 and I-Sce1 (+ SceI) plasmids and then treated with DMSO (upper panel) or the CDK12 inhibitor THZ531 for 24 h (lower panel). The fixed cells were processed to detect the γH2A. X signal by immunofluorescence. The bar graph shows the mean of the percentage of cells showing MS2 reporter gene transcription (evidenced by the co-localisation of the YFP-MS2 signal with pCherry-tTA-ER) in two independent experiments.

### Definitions

Unless otherwise defined, all technical and scientific terms used herein have the same meaning commonly understood by a person skilled in the art to which this invention belongs. Although many methods and materials similar or equivalent to those described herein may be used in practising or testing the present invention, the preferred methods and materials are described below. Unless otherwise indicated, the techniques described herein for use with the invention are standard methodologies well known to persons of ordinary skill in the art.

The term "in proximity of" when referring to the position of a DNA damage relative to genomic loci means a DNA strand break positioned within a 3 kilo-base region upstream or downstream of the transcription start site of a gene. This term also refers to all those strand-breaking events that occur within the transcribed portion of the genomic locus (gene).

The term "DNA damage events" means breaks in the double or single strand of DNA caused by replicative stress. The same phenomenon is also referred to by the expression "DDR foci" (DNA Damage Response).

### Detailed Description of the Invention

According to the present invention, cyclin-dependent kinase 12 (CDK12, cyclin-dependent kinase 12) has been found to be selectively recruited to DNA breaks that are located within transcribed loci. Therefore, it is possible to use CDK12 as a marker for DNA damage in proximity of transcribed genomic loci in a sample.

The sample is preferably a human cell sample and in particular a single human cell.

CDK12 is thus advantageously used to diagnose or classify tumours, assess their progression or highlight the sensitization of the tumour to a given therapy.

According to the present invention there is also provided a method for identifying and/or quantifying in a sample DNA damage events associated to actively transcribed genomic loci. The method comprises detecting the CDK12 protein in proximity of the aforesaid genomic loci. Preferably this is done at the level of a single cell or a single molecule.

The method according to the invention is based on detecting the localisation of the CDK12 protein at these damaged loci either by immunofluorescence assays or by using ectopic expression of chimeric proteins derived from the fusion of CDK12 with fluorescent proteins. These assays allow the detection of CDK12 in proximity of the DDR (DNA Damage Response) foci, in both live and processed single cells. The assay is selective because it does not detect DNA damage at loci of genomic regions that are not transcribed.

The method makes it possible to measure OI-RS characterised by collision between transcription and DNA replication in tumour cells. The assay can also be used for therapeutic protocol selection based on molecular analysis of tumours and as cellular assays for genetic and pharmacological screening.

The use of a single marker or assay allows to identify the presence of DNA breaks in transcribed genomic loci and represents an efficient way to identify the collision between DNA transcription and replication and replicative stress.

In an embodiment not according to the invention, the CDK12 protein is detected by binding to a CDK12-specific antibody which in turn binds a labelled secondary antibody and visualised by microscopy. In this way, CDK12 can be identified at the single cell level and at the single locus level.

In an alternative embodiment according to the invention, the CDK12 protein is detected in proximity of the aforesaid genomic loci:
- binding a primary antibody specific for CDK12 to CDK12 and a primary antibody specific for γH2AX (H2A histone family member X) to γH2AX, the two primary antibodies being derived from distinct animal species;
- binding the two primary antibodies to corresponding secondary antibodies both conjugated with DNA oligonucleotides designed so that, when γH2AX and CDK12 are in proximity, the oligonucleotides are ligated by a ligase to generate a template for a rolling circle amplification (RCA);
- performing a rolling circle amplification by means of a polymerase;
- detecting the amplification products of the rolling circle amplification;
- correlating the presence and/or the amount of rolling circle amplification products with the proximity of γH2AX and CDK12.

Preferably, the rolling circle amplification is performed by fluorescently labelled probes and the amplification products are detected and/or quantified by traditional microscopy or an automatic fluorescence reading system.

Both of the above embodiments enable localised CDK12 to be detected in subnuclear structures that correspond to transcribed loci that are damaged and the number of events (transcribed loci in which DNA is damaged) for each positive cell. The method also allows to identify absolute signals (number of CDK12 foci/nucleus) or relative signals (CDK12 signal intensity per nucleus area). In the case of relative signals, untreated cells or control tissues can be used to impose signal thresholds.

In a further alternative embodiment not according to the invention, the CDK12 protein is comprised in a fusion protein with a fluorescent protein or a halotag and the fusion protein is a stable or transient ectopically expressed protein.

The following describes the steps of the method for identifying and/or quantifying in a sample DNA damage events associated to actively transcribed genomic loci according to the present invention with particular reference to the type of sample used. As mentioned above, there are three embodiments of the method that can be used depending on user's needs and characteristics of the tissue to be analysed. The three protocols are based respectively:
- on detection by means of an antibody;
- on detection by means of two antibodies;
- on antibody-free detection.

The first step consists of the preconditioning of the biological material (optional in the first two methods and necessary instead for the antibody-free detection).

Cells or tissues can be engineered by standard molecular biology techniques (viral transfection or transduction) to express chimeric proteins derived from CDK12-cDNA fusion with a protein-based fluorescent label such as a fluorescent protein (mCHERRY, RFP, EGFP, EYP, others...) or HaloTAG. This allows the direct visualisation of CDK12 recruitment to DNA-damaged transcribed loci.

The second step consists in the treatment of the biological material.

In the case of fixed tissues, they can be included in paraffin for subsequent sectioning and slide preparation. Alternatively, fixed tissues can be frozen embedded in a cryoprotective solution. The tissues can then be sectioned to prepare the slides for microscopy. Deparaffinised tissues or cryo-preserved tissue sections are treated with standard procedures in order to recover the antigen, reduce the non-specific antibody binding (blocking or saturation) and the removal of endogenous peroxidase activity.

In the case of fixed cells, the isolated cells are fixed using a cross-linking agent.

In the case of engineered live cells and tissues, as described for the first step, cells and tissues are kept in a culture medium to preserve their integrity and viability.

The third step consists of signal detection.

In the case of fixed cells and tissues and detection by single marker, the cells or tissue slices are permeabilised and incubated with a CDK12 specific antibody. After removing the unbound anti-CDK12 antibody by washing, the cells or the slices are incubated with a secondary antibody conjugated with a fluorescent marker or with secondary antibodies for immunohistochemical detection (direct or indirect methods). The signals are acquired by standard microscopy, at the level of a single cell.

In the case of fixed cells and tissues and detection by double marker by proximity ligation assay (PLA). After permeabilisation of the cells or of the tissues and standard saturation procedures to prevent non-specific binding of antibodies, the cells or tissues are incubated with two primary antibodies specific for yH2AX and CDK12, respectively. The two antibodies have to be generated in two different species in order to be recognised by two different secondary antibodies. After removing the unbound primary antibodies by washing, the cells or the tissues are incubated with two different secondary antibodies (probes) conjugated with DNA oligos necessary to generate the signal. If the epitopes of the target proteins (γH2AX and CDK12) are close, the DNA oligos of the probes can be ligated by a DNA ligase and create a template for rolling circle amplification (RCA). Next, DNA polymerase is added for the RCA, generating a single-stranded sequence that can be labelled with detection probes to enable signal localisation. The resulting spots (foci), localised in the nuclei, can be easily detected and visualised by standard fluorescence microscopy.

In the case of live cells and tissues, the localisation of CDK12 on damaged DNA can be visualised in fixed or live cells by direct fluorescence microscopy.

In the fourth step, the signal is processed and analysed.

In the case of fixed cells or tissues and detection by single marker, with regard to signal identification, depending on the cause of DNA damage, foci or a more widespread nuclear staining can be expected to be detected. The images of the treated slides can be analysed with semi-automated procedures and dedicated software (imageJ) for the quantification of the signals at the population level (% of cells showing a positive signal, quantified as an average signal throughout the nucleus) or at the level of a single cell in order to detect the focal signals (number of foci/nucleus) or the average signal intensity in the nuclei (in the case of more widespread signals).

In the case of fixed cells and tissues and detection by double marker through proximity ligation assay (PLA), the sensitivity that can be obtained is greater, as is the quantitative power (since PCR amplification increases detection of nuclear foci) and its selectivity is greater compared to single-marker detection methods. This method allows quantitative detection of endogenous CDK12 foci with low background noise. Therefore, an absolute signal threshold can be set to identify cells under replicative stress. Based on the experience with X-ray-irradiated in vitro cultured cell lines, normal cells show an average of 2 (+/- 2) PLA-positive nuclear foci. Generally, cells with more than 5 foci are considered subject to transcription-coupled DNA damage. The images can be analysed with semi-automated procedures and dedicated software (imageJ) for the quantification of the signals at the level of single cells (number of labelled areas per cell) or at the population level (% of cells showing labelled areas).

In the case of live cells and tissues, images can be analysed with semi-automated procedures and dedicated software (imageJ) for the quantification of the signals at the population level (% of cells showing a positive signal) or at the level of a single cell (number of signals or total signal intensity in nuclei).

In cases where signals are quantified as the number of nuclear foci, the sensitivity of this method will allow the detection with a single-locus detection limit, whereby each focus indicates a site where CDK12 associated to a damaged genomic locus is detected. Therefore, the number of foci detected will reflect the number of genomic loci that suffer DNA damage associated to replicative transcription/stimulation.

A detailed description of the three embodiments follows.

### 1. ANTIBODY-BASED DETECTION

The procedure is based on established protocols for antibody-based detection of cell epitopes in cells and tissues, using standard immunofluorescence or immunohistochemistry methods. In short, the method consists of the following steps:
1) cells (from liquid tissue such as blood) or tissue are sampled and fixed using a cross-linking agent (e. g. formaldehyde or para-formaldehyde). Tissues can be included in paraffin for subsequent sectioning and slide preparation.
   Alternatively, fixed tissues embedded in a cryoprotective solution can be frozen.
2) Tissues can be sectioned to prepare the slides for microscopy.
3) Deparaffinised tissues or cryo-preserved tissue sections are treated with standard procedures in order to recover the antigen and permeabilise the cells, to reduce the non-specific antibody binding (blocking or saturation) and the removal of endogenous peroxidase activity.
4) Cells and tissues (slides) are incubated with a CDK12 specific antibody.
5) After removing the unbound anti-CDK12 antibody by washing, the cell or tissue slides are incubated with a secondary antibody conjugated with a fluorescent marker or with secondary antibodies for immunohistochemical detection (direct or indirect methods).
6) With regard to signal identification, depending on the cause of DNA damage, foci or a more widespread nuclear staining can be expected to be detected. The images of the treated slides can be analysed with semi-automated procedures and dedicated software (imageJ) for the quantification of the signals at the population level (% of cells showing a positive signal, quantified as an average signal throughout the nucleus) or at the level of a single cell to detect the focal signals (number of foci/nucleus) or the average signal intensity in the nuclei (in the case of more widespread signals). In cases where signals are quantified as the number of nuclear foci, the sensitivity of this method will allow the detection with a single-locus detection limit, whereby each focus indicates a site where CDK12 associated with a damaged genomic locus is detected. Therefore, the number of foci detected will reflect the number of genomic loci that suffer DNA damage associated to replicative transcription/stimulation.

### 2. ANTIBODY-BASED DETECTION (DUAL-MARKERS, PLA-ASSAY)

This procedure is based on the method called proximity ligation assay (PLA-assay), a procedure that uses antibody-based technology and PCR to visualise the physical proximity of biomolecules. Using this method, it is possible to detect the proximity of CDK12 to a DDR marker such as γH2AX. The advantage of this method is the increased sensitivity, the higher quantitative power (since PCR amplification increases the detection of nuclear foci) and its higher selectivity compared to the methods of detection of the single markers. This method allows quantitative detection of endogenous CDK12 foci with low background, so an absolute signal threshold can be set to identify cells subject to replicative stress. With X-ray-irradiated in vitro cultured cell lines, normal cells show an average of 2 (+/- 2) PLA-positive nuclear foci.

In short, the method consists of the following steps:
1) The cells are fixed using a cross-linking agent. Tissues can be included in paraffin for subsequent sectioning and slide preparation. Alternatively, fixed tissues embedded in a cryoprotective solution can be frozen.
2) Tissues can be sectioned to prepare the slides for microscopy.
3) Deparaffinised tissues or cryo-preserved tissue sections are treated with standard procedures in order to recover antigen, permeabilise cells, reduce non-specific antibody binding (blocking or saturation) and the removal of endogenous peroxidase activity.
4) The cells are incubated with two primary antibodies specific for γH2AX and CDK12 (the two antibodies must be produced in two different species).
5) After removing the unbound antibodies by washing, the cells are incubated with two different secondary antibodies (probes) conjugated with DNA oligos necessary to generate the signal. If the epitopes of the target proteins are close, the DNA oligos of the probes can be ligated and create, via a DNA ligase enzyme, a template for rolling circle amplification (RCA).
6) Next, DNA polymerase for RCA is added, generating a single-stranded sequence that can be labelled with detection probes to enable signal localisation.
7) The resulting labelled areas, localised in the nuclei, can be easily detected and visualised by standard fluorescence microscopy. The images can be analysed by semi-automated procedures and dedicated software (imageJ) for the quantification of the signals at the level of single cells (number of labelled areas per cell) or at the population level (% of cells showing labelled areas). In cases where signals are quantified as the number of nuclear foci, the sensitivity of this method allows the detection with a single-locus detection limit, whereby each focus indicates a site where CDK12 associated with a damaged genomic locus is detected. Therefore, the number of foci detected will reflect the number of genomic loci that suffer DNA damage associated to replicative transcription/stimulation.

### 3. ANTIBODY-FREE DETECTION (detection based on labelled CDK12)

Cells or tissues can be engineered to express chimeric proteins derived from the fusion of CDK12-cDNA with a protein-based fluorescent marker such as a fluorescent protein (mCHERRY, RFP, EGFP, EYP, others. . . ) or HaloTAG. This allows the direct visualisation of CDK12 recruitment to DNA-damaged transcribed loci.

Briefly, the method comprises the following steps.
1) Cloning of the chimeric cDNA encoding the CDK12-marker fusion protein in an expression vector, suitable for transient or stable expression (i. e. viral vectors) of the CDK12-marker in cells.
2) Target cells or tissues can be transfected with expression vectors or transduced with viral particles encoding CDK12-markers.
3) The localisation of CDK12 on damaged DNA can be visualised in fixed or live cells by fluorescence microscopy.
4) The images can be analysed with semi-automated procedures and dedicated software (imageJ) for the quantification of the signals at the population level (% of cells showing a positive signal) or at the level of a single cell (number of signals or total signal intensity in nuclei) . In cases where signals are quantified as the number of nuclear foci, the sensitivity of this method will allow the detection with a single-locus detection limit, whereby each focus indicates a site where CDK12 associated with a damaged genomic locus is detected. Therefore, the number of foci detected will reflect the number of genomic loci that suffer DNA damage associated to replicative transcription/stimulation.

### Examples

Below is a list of conditions and experiments demonstrating the possibility of visualising and measuring CDK12 recruitment to regions of genomic DNA adjacent to transcribed genomic loci. These observations demonstrate the reliability of the method for detecting the association of CDK12 with genomic regions possessing the following characteristics:
(1) presence of a DNA break in the double strand (DSB); (2) activation of the DNA damage response (DDR) system; (3) regions where transcription takes place.

This experimental evidence was organised into four examples:
Example 1: experimental evidence demonstrating the localisation of CDK12 in damaged genomic and transcribed sites.
Example 2: experimental evidence demonstrating the localisation of CDK12 in nuclear foci where DDR is activated (DDR-foci) is not dependent on the catalytic activity of CDK12.
Example 3: experimental evidence that CDK12 is not a general marker of DDR-foci, but is selectively recruited by those DDR processes that are regulated by PARP1.
Example 4: experimental evidence demonstrating that CDK12 regulates the transcriptional activity of those genes that are positioned proximally to regions of damaged DNA (DSB).

### Example 1: CDK12 is recruited in DDR sites proximal to transcribed genes and its recruitment to these sites is transcriptionally dependent.

*CDK12 is transiently detected in X-ray-generated DNA damaged sites.*

To assess the recruitment of CDK12 at DNA-damaged sites (single- and double-strand breaks), the cells were irradiated with X-rays and analysed at successive times after irradiation in order to detect the proximity between CDK12 and DDR foci. DDR foci were detected with an antibody specific for the phosphorylated form of H2Ax (γH2Ax). The proximity was assessed by Proximity Ligation Assay (PLA), a PCR-based technique that allows the spatial resolution of protein complexes on a nanoscale. The specificity of the PLA signal was assessed in the cells in which the expression of CDK12 was silenced by specific shRNAs. A high number of nuclear PLA spots was detected in the cells immediately after irradiation, while non-irradiated cells showed signal levels close to background noise.

In addition, after irradiation, a high number of PLA signal-positive nuclear foci was also detected in U2OS cells transfected with the chimeric Flag-CDK12 cDNA (Figure 1).

*CDK12 is recruited at damaged DNA sites induced by laser micro-irradiation.*

U2OS cells were transfected with a vector encoding mCherry-CDK12 or mCherry (as a negative control). The cells were micro-irradiated with a laser beam to induce DNA breaks, so that these breaks were confined to selected nuclear areas. Live confocal microscopy analyses showed rapid recruitment of mCherry-CDK12 in the damaged areas. In contrast, mCherry showed no selective recruitment in the damaged areas, but only a slight recovery of the signal due to molecular diffusion (Figure 2).

*CDK12 can be detected at the level of a single DSB and of a single cell in a DSB adjacent to a transcribed gene.*

U2OS-TRE-I-Sce1 cells were used (Ui et al., 2015), which were genetically engineered with a reporter system that allows the generation of DNA double-strand breaks in a programmable manner, the conditional expression of a reporter gene for the transcriptional activity (proximal to the DSB) and the analysis of nascent RNA synthesis, encoded by the transcriptional reporter. The reporter system comprises a single restriction site that is recognised by a DNA endonuclease (Sce-1). The Sce-1 restriction site is followed by a tetracycline-responsive promoter and a reporter gene whose expression can be visualised using an RNA-binding chimeric fluorescent protein (YFP-MS2). U2OS-TRE-I-Sce1 cells were transfected with plasmids encoding (i) YFP-MS2 (a chimeric gene produced by MS2 cDNA fused with YFP cDNA), (ii) mCherry-CDK12 (a chimeric fusion of mCherry cDNA and CDK12 cDNA), (iii) rtTA a doxycycline-activated transcription factor and (iv) the restriction enzyme Sce-1. Transcription was activated by the addition of doxycycline while transcriptional activity was detected by visualising positive nuclear foci for YFP signal. Sce-1-induced DSBs were visualised by immunofluorescence staining of the phosphorylated form of the histone H2Ax, a marker for DNA break detection and DNA damage response (DDR) signalling pathway activation. These focal nuclear signals of γH2A. X are called DDR foci. After transcription activation and DSB induction by Sce-1 endonuclease, a single focal signal for CDK12 (CDK12-focus) was detected in the nucleus. Analyses show how CDK12 foci overlap with DDR signals (foci of γH2A. X), thus demonstrating that CDK12 is recruited at damaged DNA regions. In the absence of Sce-1, neither DDR foci nor CDK12 foci were detected. As expected, the induction of a DSB inhibits the transcription of the neighbouring gene, as shown by the lack of a positive focus for YFP at the DDR foci (Figure 3).

### CDK12 is recruited at endogenous transcription-associated DDR sites

RPE-iCUT cells were used for site-specific DSB induction in selected endogenous genomic loci. These are immortalised retinal pigment epithelial cells that have been genetically modified to allow the expression and the conditional activation of the gene encoding for the CRISPR-CAS9 nuclease. Cells were transfected with mCherry-CDK12 and a set of sgRNAs (Alt-R-gRNA) designed to induce DSB in three expressed genes (MCM2, IFRD2 and POLR1B), in two regions upstream of their transcription start site (TSS), or designed to induce DSB in two regions distal to transcribed loci. Genomic editing (i. e. DSBs) was assessed by detecting foci of γH2A. X, while CDK12 recruitment was assessed by detecting the mCherry signal. The results show that while CDK12 foci were detected at DDR foci proximal to the promoters, the distal DDR foci do not show co-localisation with CDK12. These observations indicate that CDK12 is preferentially recruited at damaged sites near transcribed genes (Figure 4).

*Recruitment of CDK12 to a programmed DSB in the U2OS-TRE-I-Sce1 cell line requires active transcription of the proximal locus.*

U2OS-TRE-I-Sce1 cells were transfected with plasmids encoding (i) YFP-MS2 (a chimeric gene produced by MS2 cDNA fused with YFP cDNA), (ii) mCherry-CDK12 (a chimeric fusion of mCherry cDNA and CDK12 cDNA), (iii) rtTA a doxycycline-activated transcription factor and (iv) the restriction enzyme Sce-1. Transcription was activated by the addition of doxycycline and monitored by detecting positive nuclear foci for YFP signal. Sce-1-induced DSBs were visualised by immunofluorescence analysis of the phosphorylated form of the histone H2Ax (γH2A. X), a marker for the detection of DNA breaks and the activation of the DNA Damage Response (DDR) signalling pathway. CDK12 foci superimposed on DDR foci were only detected when the rtTA transcription factor was activated by doxycycline. The lack of rtTA induction did not affect the formation of Sce-1-dependent DDR foci, but prevented the formation of CDK12 foci. On the other hand, transcription activation, without DSB induction, did not trigger CDK12 foci formation (data not shown). Overall, this indicates that the recruitment of CDK12 to a programmed DSB depends on the transcriptional status of the neighbouring locus (Figure 5).

### Recruitment of CDK12 at DNA-damaged sites induced by laser depends on the activity of RNA-pol2

U2OS cells were transfected with a vector encoding mCherry-CDK12. Cells were micro-irradiated with a laser beam to induce DNA breaks confined to appropriately selected nuclear areas.

In order to inhibit RNA-pol2, the cells were pre-treated with either (i) triptolide (a TFIIH inhibitor that blocks the start of transcriptional) or (ii) DRB (5,6-dichlorobenzimidazole riboside, a DSIF inhibitor that prevents transcriptional elongation). The inhibition of RNA-Pol2 activity by triptolide or DRB prevents the recruitment of mCherry-CDK12 at damaged areas, thus indicating that the simultaneous presence of active transcription and DNA damage is necessary for the localisation of CDK12 in these loci (Figure 6).

*The recruitment of CDK12 to programmed DSBs in the U2OS-TRE-I-Sce1 cell line requires RNA-pol2 activity.*

U2OS-TRE-I-Sce-1 cells were transfected with plasmids encoding (i) YFP-MS2 (a chimeric gene produced by the MS2 cDNA fused with the YFP cDNA), (ii) mCherry-CDK12 (a chimeric fusion of mCherry and CDK12), (iii) rtTA (a doxycycline-activated transcription factor) and (iv) the restriction enzyme Sce-1. Transcription was activated by the addition of doxycycline and monitored by detecting subcellular positive nuclear foci for YFP signal. Sce-1-induced DSBs were visualised by immunofluorescence staining specific for the phosphorylated form of histone H2Ax (γH2A. X), a marker for DNA break detection and DNA damage response (DDR) signalling pathway activation. In order to inhibit RNA-pol2, the cells were pre-treated with either (i) triptolide (a TFIIH inhibitor blocking transcriptional onset) or (ii) DRB (5,6-dichlorobenzimidazole riboside, a DSIF complex inhibitor preventing the transcriptional elongation process). The inhibition of RNA-Pol2 activity by triptolide or DRB prevents the recruitment of mCherry-CDK12 at damaged areas, thus indicating that the simultaneous presence of active transcription and DNA damage is necessary for the localisation of CDK12 in these loci (Figure 7).

*The recruitment of CDK12 at endogenous DDR sites is dependent on RNA-pol2 activity.*

For the site-specific induction of a DSB at selected endogenous genomic loci, we used RPE-iCUT cells: immortalised retinal epithelial cells engineered for CRISPR-CAS9 expression and conditional activation. These cells were transfected with mCherry-CDK12 and a set of sgRNAs (Alt-R-gRNA) designed to induce DBS in three expressed genes (MCM2, IFRD2 and POLR1B), in two regions upstream of their site of transcriptional onset (TSS), or designed to induce DSB in two genomic regions distal to transcribed loci. The induction of DSBs by CRISPR-Cas9 was assessed by immunofluorescence of γH2A. X, while the recruitment of CDK12 to DNA-damaged sites was assessed by microscopy analysis of mCherry signals. While CDK12 foci were detected at DDR foci proximal to promoters, RNA-pol2 inhibition by DRB reduced its recruitment to damaged foci, thus indicating that CDK12 binding to damaged DNA requires RNA-pol2 activity. As a control, DSBs induced in regions distal to transcribed loci show low levels of CDK12 recruitment (close to the background signal), which are not altered by RNA-pol2 inhibitors (Figure 8).

### Example 2: Recruitment of CDK12 at DDR sites is independent of its catalytic activity

*Recruitment of CDK12 at DNA-damaged sites by laser micro-irradiation is independent of CDK12 catalytic activity*

U2OS cells were transfected with a vector encoding for mCherry-CDK12. Cells were micro-irradiated with a laser beam to induce DNA breaks confined to selected nuclear areas. In order to inhibit CDK12, the cells were pre-treated with THZ531, a covalent inhibitor of CDK12. THZ531 did not affect the recruitment of CDK12 at DDR foci, thus indicating that kinase activity is not required for this process (Figures 9A and 9B).

### Example 3: the recruitment of CDK12 at DDR foci is independent of the signalling mechanisms regulated by ATR and ATM, but is dependent on PARP1 activity.

*Recruitment of CDK12 to laser-induced DSBs is not dependent on ATR and ATM activity.*

U2OS cells were transfected with a vector encoding for mCherry-CDK12. The cells were micro-irradiated with a laser beam to induce DNA breaks in selected nuclear areas. To inhibit the DDR response, the cells were pre-treated with an ATM inhibitor (KU-55933) or an ATR inhibitor (VE-821). Neither of the two inhibitor affected the localisation of CDK12 at the damaged sites, thus indicating that this process is not dependent on the apical regulators of the DNA damage response (Figures 10A and 10B).

*The recruitment of CDK12 at DNA-damaged sites is dependent on PARP1 activity.*

U2OS cells, transfected with a vector encoding for mCherry-CDK12, were micro-irradiated with a laser beam to induce DNA breaks confined to selected nuclear areas. To inhibit PARP1, the cells were pre-treated with Olaparib and Veliparib. Both inhibitors inhibited the localisation of CDK12 at damaged DNA sites, thus indicating that this process is dependent on PARP1 activity (Figures 11A and 11B).

### Example 4: CDK12 is required to repress transcription at the damaged gene loci

### Gene silencing of CDK12 induces global de-repression of transcription

U2OS cells, transfected with siRNAs that silence CDK12 expression, were irradiated with X-rays to damage the DNA. The synthesis of nascent RNA was monitored by metabolic labelling with 5-EU followed by immunofluorescence analysis to detect EU incorporation into nuclei. DRB, an RNA-pol2 inhibitor, was used as a control in order to reduce nascent RNA synthesis. CDK12 silencing increased nascent RNA synthesis both under normal conditions and in irradiated cells, thus indicating its role as a general inhibitor of transcription (Figure 12).

### CDK12 silencing prevents inhibition of the transcription of damaged genes

U2OS-TRE-I-Sce1 cells were transfected with plasmids encoding (i) YFP-MS2 (a chimeric gene produced by the MS2 cDNA fused with the YFP cDNA), (ii) mCherry-tTA-ER (a fusion of the transcription factor tTA that is activated by hydroxytamoxifen (OHT)) and (iii) the restriction enzyme Sce1. Transcription was activated by addition of OHT and monitored by detecting positive nuclear foci for YFP signal. Sce1-induced DSBs were visualised by immunofluorescence staining of the phosphorylated form of the histone H2Ax (γH2A. X), a marker for DNA break detection and DNA damage response (DDR) signalling pathway activation. As expected, the presence of a DSB inhibits the transcription of the proximal reporter gene. Conversely, when CDK12 expression is silenced, the transcription of the reporter gene occurs despite the presence of DSB. This result indicates that CDK12 is required for the transcriptional inhibition of damaged genes (Figure 13).

*The pharmacological inhibition of CDK12 prevents the repression of the transcription of damaged genes*
U2OS-TRE-I-Sce1 cells were transfected with plasmids encoding (i) YFP-MS2 (a chimeric gene produced from MS2 cDNA fused with YFP cDNA), (ii) mCherry-tTA-ER and (iii) the restriction enzyme Sce-1. Transcription was activated by addition of OHT and monitored by detecting positive nuclear foci for YFP signal. Sce1-induced DSBs were visualised by immunofluorescence staining of the phosphorylated form of the histone H2Ax (γH2A. X), a marker for DNA break detection and DNA damage response (DDR) signalling pathway activation. Although the damage to proximal DNA of the promotor altered MS2 locus transcription, CDK12 inhibition by THZ531 reactivated MS2 reporter expression, thus indicating that CDK12 prevents the transcriptional activation on damaged genes (Figure 14).

### Advantages

A single marker test has the great advantage of being practical, easily adaptable to specific test conditions, scalable for high-throughput/high-information content microscopic analysis and can be easily integrated into automated sample processing for clinical/industrial use.

## Claims

1. A method for identifying and/or quantifying in a sample the DNA damage events associated to actively transcribed genomic loci comprising detecting the CDK12 protein in proximity of these genomic loci by:
- binding a primary antibody specific for CDK12 to CDK12 and a primary antibody specific for γH2AX (H2A histone family member X) to γH2AX, the two primary antibodies being derived from distinct animal species;
- binding the two primary antibodies to corresponding secondary antibodies both conjugated with DNA oligonucleotides designed so that, when γH2AX and CDK12 are in proximity, the oligonucleotides are ligated by a ligase to generate a template for a rolling circle amplification (RCA);
- performing a rolling circle amplification by means of a polymerase;
- detecting the amplification products of the rolling circle amplification;
- correlating the presence and/or the amount of rolling circle amplification products with the proximity of yH2AX and CDK12.

2. The method according to claim 1, wherein the sample is a single cell.

3. The method according to claim 1 or 2, wherein the rolling circle amplification is performed by fluorescently labelled probes and the amplification products are detected and/or quantified by traditional fluorescence microscopy or an automatic fluorescence reading system.

## Patentansprüche

1. Verfahren zum Identifizieren und/oder Quantifizieren von DNA-Schadensereignissen in einer Probe, die mit aktiv transkribierten genomischen Loci verbunden sind, umfassend das Nachweisen des CDK12-Proteins in der Nähe dieser genomischen Loci durch:
- Binden eines primären Antikörpers, der spezifisch für CDK12 ist, an CDK12 und eines primären Antikörpers, der spezifisch für γH2AX (H2A Histon-Familienmitglied X) ist, an γH2AX, wobei die beiden primären Antikörper von unterschiedlichen Tierarten stammen;
- Binden der beiden primären Antikörper an entsprechende sekundäre Antikörper, die beide mit DNA-Oligonukleotiden konjugiert sind, die so konzipiert sind, dass, wenn γH2AX und CDK12 in der Nähe sind, die Oligonukleotide durch eine Ligase ligiert werden, um eine Vorlage für eine Rolling-Circle-Amplifikation (RCA) zu erzeugen;
- Durchführen einer Rolling-Circle-Amplifikation mit Hilfe einer Polymerase;
- Nachweisen der Amplifikationsprodukte der Rolling-Circle-Amplifikation;
- Korrelieren des Vorhandenseins und/oder der Menge der Rolling-Circle-Amplifikationsprodukte mit der Nähe von γH2AX und CDK12.

2. Verfahren nach Anspruch 1, wobei die Probe eine einzelne Zelle ist.

3. Verfahren nach Anspruch 1 oder 2, wobei die Rolling-Circle-Amplifikation mit fluoreszenzmarkierten Sonden durchgeführt wird und die Amplifikationsprodukte durch herkömmliche Fluoreszenzmikroskopie oder ein automatisches Fluoreszenz-Lesesystem nachgewiesen und/oder quantifiziert werden.

## Revendications

1. Procédé d'identification et/ou de quantification dans un échantillon des événements de dommages à l'ADN associés à des locus génomiques activement transcrits, comprenant la détection de la protéine CDK12 à proximité de ces locus génomiques en :
- liant un anticorps primaire spécifique de CDK12 à CDK12 et un anticorps primaire spécifique de γH2AX (membre X de la famille des histones H2A) à yH2AX, les deux anticorps primaires étant dérivés d'espèces animales distinctes ;
- liant les deux anticorps primaires aux anticorps secondaires correspondants, tous deux conjugués à des oligonucléotides d'ADN conçus de telle sorte que, lorsque γH2AX et CDK12 sont à proximité, les oligonucléotides sont ligaturés par une ligase pour générer une matrice pour une amplification en cercle roulant (RCA) ;
- réalisant une amplification en cercle roulant au moyen d'une polymérase ;
- détectant les produits d'amplification de l'amplification en cercle roulant ;
- corrélant la présence et/ou la quantité de produits d'amplification en cercle roulant avec la proximité de yH2AX et de CDK12.

2. Procédé selon la revendication 1, l'échantillon étant une cellule unique.

3. Procédé selon la revendication 1 ou 2, l'amplification en cercle roulant étant réalisée par des sondes marquées par fluorescence et les produits d'amplification étant détectés et/ou quantifiés par microscopie à fluorescence traditionnelle ou par un système de lecture automatique de la fluorescence.
